# EUROPEAN PATENT APPLICATION

(11) **EP 1 803 467 A1**
(43) Date of publication of application: **04.07.2007**
(21) Application number: 05774571.3
(22) Date of filing: 25.08.2005
(51) Int. Cl.: A61K 41/00, A61K 45/00, A61K 47/30, A61K 47/48, A61P 35/00, A61P 43/00, A61F 7/00

(54) **COMPOSITE FINE PARTICLES AND PROCESS FOR PRODUCING THE SAME**

(30) Priority: 25.08.2004 JP 2004245844
(71) Applicant: Kagoshima University, Kagoshima-shi, Kagoshima 890-8580 (JP)
(72) Inventor: AOYAGI, Takao, Kagoshima-shi, Kagoshima 8900055 (JP)
(74) Representative: Vossius & Partner
(86) International application number: PCT/JP2005/015451
(87) International publication number: WO 2006/022340

(57) **Abstract**

A temperature-responsive polymer (hydrated graft structure) layer (2) composed of acrylamide derivative or the like exhibiting sensitive temperature-responsivity is immobilized to a magnetic particulate (1) composed of iron oxide or the like with a covalent bond. Further, an anticancer agent (3) is dispersed in the temperature-responsive polymer (2). The anticancer agent is ionically bonded to the temperature responsive polymer (hydrated graft structure).

## Description

### Technical Field

The present invention relates to a composite particulate suitable for a cancer treatment and a method of manufacturing the same.

### Background Art

In recent years, a research aiming at establishment of thermotherapy for cancer using a magnetic particulate has been carried out (for instance, non-Patent Document 1). A temperature-responsive medicine-releasing liposome (for instance, non-Patent Document 2) and a magnetic particulate covered with a phospholipid double layer (for instance, non-Patent Document 3) have been reported regarding cancer treatment.

However, the conventional thermotherapy using magnetic particulates alone makes it possible to heat locally, there arises a disadvantage that a cancer cell bears a thermal resistance due to production of heat shock protein. Whereas an associated liposome formed by a phospholipid molecule has a disadvantage of being unstable in a human body because it is formed only by association of phospholipid.

[Patent Document 1] Japanese Patent Application Laid-open 2002-223793
[Patent Document 2] Japanese Patent Application Laid-open 2000-212144
[non-Patent Document 1] Masashige Shinkai, Kousuke Ueda, Shinji Ohtsu, Hiroyuki Honda and Takeshi Kobayashi, Japanese Journal of Cancer Research, Vol. 90, 699-704 (1999)
[non-Patent Document 2] M.B. Yatvin, J.N. Weinstein, W.H. Dennis, R. Blumenthal Science Vol. 202 1290-1293 (1978)
[non-Patent Document 3] Akira Ito, Masashige Shinkai, Hiroyuki Honda and Takeshi Kobayashi, Cancer Gene Therapy, Vol. 8, 649-654 (2001)

### Summary of the Invention

An object of the present invention is to provide a composite particulate which can destroy only cancer tissue without affecting a normal tissue, and a method of manufacturing the particulate.

In order to solve the above-described problem, the present inventor has paid attention to and studied about an acrylamide derivative polymer which has a functional group possible to cause a chemical reaction, and exhibits sensitive temperature-responsivity, so as to enable an effective cancer therapy by combining thermotherapy and chemotherapy, and found it possible to compose a magnetic particulate which sensitively responds to a temperature based on voluntarily heat generation in a magnetic field of the magnetic particulate by connecting a magnetic particulate and the polymer. In addition, the present inventor has found it possible to execute effective chemotherapy even when a particulate other than the magnetic particulate, for instance, a metal particulate or an active carbon particulate is used, provided that the above-described polymer is used, and has come up with the present invention.

A composite particulate according to the present invention includes a particulate, and a hydrated graft structure connected to the surface of the particulate and composed of a temperature-responsive polymer and water, in which the general formula of the temperature-responsive polymer is expressed as follows. 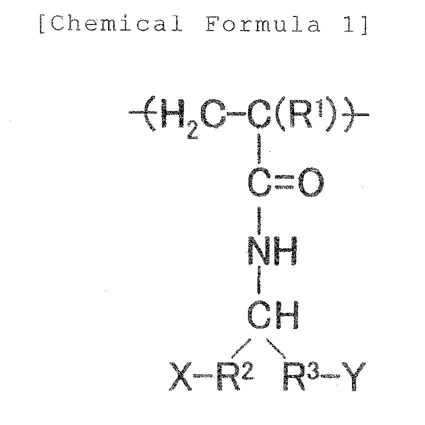 (where R¹ represents a hydrogen atom, or a straight chain or a branched alkyl group with a carbon number of 1 to 4,
R² represents a straight chain or a branched alkyl group with a carbon number of 1 to 4,
R³ represents a methylene group with a carbon number of 1 to 6,
X represents a hydrogen atom, an amino group, a hydroxyl group, a halogen atom, a carboxyl group or - COOR⁴ (where R⁴ represents a straight chain or a branched alkyl group with a carbon number of 1 to 6, a phenyl group, a substituted phenyl group, a benzyl group or a substituted benzyl group), and
Y represents an amino group, a hydroxyl groups a halogen atom, a carboxyl group or -COOR⁴ (where R⁴ represents a straight chain or a branched alkyl group with a carbon number of 1 to 6, a phenyl group, a substituted phenyl group, a benzyl group or a substituted benzyl group). R² and R³ may be united together and form a ring.)

A method of manufacturing a nano-level composite body according to the present invention includes the steps of introducing a functional group on the surface of a magnetic particulate using a silane coupling reagent, connecting a hydrated graft structure composed of a temperature-responsive polymer and water on the surface of the magnetic particulate with a covalent bond via the functional group, and connecting the hydrated graft structure and a compound with an ionic bond.

An agent according to the present invention contains the composite particulate as an effective component.

An apparatus for thermo- and chemotherapy according to the present invention includes a locally injector provided with a locator specifying a position of a cancer cell and an alternating-current magnetic field applying portion applying an alternating-current magnetic field at 100 kHz to 10 MHz to the composite particulates locally located by the locally injector.

### Brief Description of the Drawings

Fig. 1 is a cross sectional view showing a composite particulate according to an embodiment of the present invention;
Fig. 2 is a TEM photo showing an image of a temperature-responsive polymer immobilizing magnetic particulate using a temperature-responsive polymer No. 1;
Fig. 3 is a TEM photo showing an image of a temperature-responsive polymer immobilizing magnetic particulate using a temperature-responsive polymer No. 2;
Fig. 4 is a TEM photo showing an image of untreated magnetic particulates;
Fig. 5 is a view showing a reaction of a magnetite particulate to a silane coupling reagent;
Fig. 6A is a view showing a target of an X-ray photoelectron spectroscopy;
Fig. 6B is a graph showing an analysis result of nitrogen (N) 1s orbit;
Fig. 6C is a graph showing an analysis result of silicon (Si) 2p orbit;
Fig. 7 is a view showing a temperature-responsive polymer layer in a composite particulate;
Fig. 8A is a TEM photo of a particulate before immobilization of a polymer;
Fig. 8B is a TEM photo of a composite particulate after immobilization of a polymer;
Fig. 8C is a TEM photo of a composite particulate after immobilization of a polymer similarly to Fig. 8B;
Fig. 9A is a view showing a chemical structure of a composite particulate;
Fig. 9B is a graph showing the result of an X-ray photoelectron spectroscopy;
Fig. 10 is a graph showing a relation between immobilization of a polymer and coagulation behavior of a composite particulate;
Fig. 11 is a view showing the evaluation result of dispersibility accompanying solvent affinity of a polymer;
Fig. 12 is a graph showing a relation between temperature variation and coagulation behavior of a composite particulate;
Fig. 13 is a view showing an observation result of the change in hydrophobic nature on the surface of a composite particulate;
Fig. 14 is a view showing an observation result of the change in characteristics on the surface of a composite particulate accompanying irradiation of RF radiation;
Fig. 15 is a graph showing a confirmation result of immobilization of an anticancer agent on a composite particulate; and
Fig. 16 is a graph showing a confirmation result of release of an anticancer agent from a composite particulate.

### Detailed of the Preferred Embodiments

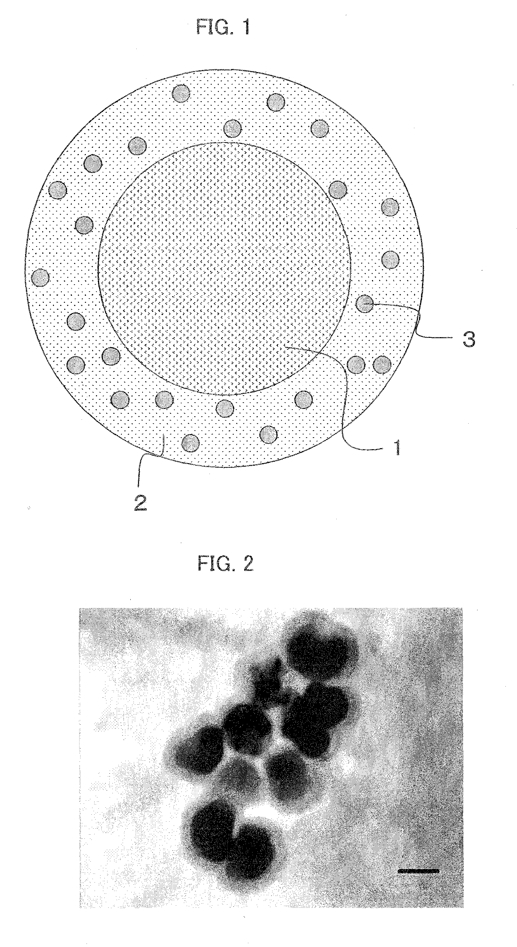

An embodiment of the present invention will be explained hereinafter. Fig. 1 is a cross sectional view showing a composite particulate according to an embodiment of the present invention.

In the present embodiment, a temperature-responsive polymer (hydrate graft structure) layer 2 composed of acrylamide derivative or the like exhibiting sensitive temperature-responsivity is immobilized to a magnetic particulate 1 composed of iron oxide or the like having a diameter of several nanometers to several hundred nanometers, more preferably, from several nanometers to two hundred nanometers with a covalent bond. In addition, in the temperature-responsive polymer layer 2, an anticancer agent 3 is dispersed. The anticancer agent 3 ionically bonds to the temperature-responsive polymer (hydrated graft structure). Note that though the anticancer agent 3 is included in the temperature-responsive polymer layer 2 for convenience in illustration in Fig. 1, it is not necessary to be included in such a manner.

When an alternating-current magnetic field is applied on the composite particulate thus structured, the magnetic particulate 1 generates heat. The temperature-responsive polymer dissolves in water when the temperature of the water is below a prescribed temperature, and becomes insoluble at a higher temperature. In other words, it becomes hydrophilic on the lower temperature side, and hydrophobic on the higher temperature side. An ionic group which does not participate with the immobilization reaction of the temperature-responsive polymer to the magnetic particulate 1 becomes dissociable on the lower temperature side and non-dissociable on the higher temperature side according to this temperature variation. Accordingly, in the present embodiment, by adjusting the above-described prescribed temperature of the temperature-responsive polymer, the anticancer agent 3 is immobilized to the magnetic particulate 1 before heat generation of the magnetic particulate 1, and the temperature-responsive polymer is made hydrophobic by causing the magnetic particulate 1 to generate heat, so that the anticancer agent 3 is disengaged.

Accordingly, when the magnetic particulate 1 is forced to generate heat so as to keep the local temperature in the neighborhood of a cancer cell at a high temperature, by locally positioning the composite particulate according to the present embodiment close to the cancer cell using a local injector or the like and by giving an alternating-current magnetic field, the temperature-responsive polymer is contracted responding to the locally high temperature. Then, the anticancer agent 3 ionically bonding to the hydrated graft structure of the temperature-responsive polymer is gradually released and disengaged, and attacks the cancer cell. In other words, a chemical treatment is given and at the same time a thermal treatment by heat generation of the magnetic particulate 1 is also given.

Thus, in the present embodiment, the temperature-responsive polymer is covalently bonded to the magnetic particulate 1, and the anticancer agent 3 is immobilized by an ionic mutual operation of a part of functional groups of the temperature-responsive polymer, so that it becomes possible to improve in affinity of a cancer tissue due to thermal effect and change into hydrophobic, and to controllably release the anticancer agent 3, responding to temperature variation, utilizing a heat generation phenomenon of the magnetic particulate 1 in an alternating-current magnetic field.

A method of manufacturing the above-described composite particulate will be explained next .

As the magnetic particulate 1, an iron oxide compound particulate such as, for instance, hematite, maghemite, magnetite, or the like, can be used, and a compound made by substituting iron atoms in these iron oxide compounds by manganese, cobalt, or the like can also be used.

As the temperature-responsive polymer composing the temperature-responsive polymer layer 2, a polymer or a copolymer containing an acrylamide derivative expressed by the following general formula (I) (Chemical Formula 2) can be used. Such a temperature-responsive polymer is disclosed in, for instance, Patent Document 2.

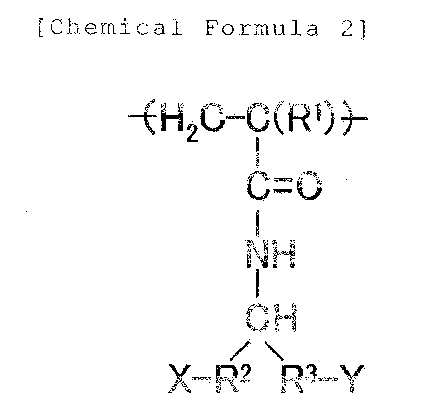

Where in the general formula (I), R¹ represents a hydrogen atom, or a straight chain or a branched alkyl group with a carbon number of 1 to 4,
R² represents a straight chain or a branched alkyl group with a carbon number of 1 to 4,
R³ represents a methylene group with a carbon number of 1 to 6,
X represents a hydrogen atom, an amino group, an hydroxyl group, a halogen atom, a carboxyl group or - COOR⁴ (where R⁴ represents a straight chain or a branched, alkyl group with a carbon number of 1 to 6, a phenyl group, a substituted phenyl group, a benzyl group or a substituted benzyl group), and
Y represents an amino group, a hydroxyl group, a halogen atom, a carboxyl group or -COOR⁴ (where R⁴ represents a straight chain or a branched alkyl group with a carbon number of 1 to 6, a phenyl group, a substituted phenyl group, a benzyl group or a substituted benzyl group). R² and R³ may be united together and form a ring.)

As an example of a straight chain or branched alkyl group with a carbon number of 1 to 4 represented by R¹ and R² composing the general formula (I), a methyl group, an ethyl group, an n-propyl group, an i-propyl group, an n-butyl group, an i-butyl group, an s-butyl group and a t-butyl group can be cited, and in addition to these, an n-pentyl group, and an n-hexyl group can also be cited.

As an example of methylen groups with a carbon number of 1 to 6 represented by R³, a monomethylene group, a dimethylene group, a trimethylene group, and a tetramethylene group can be cited, and it is preferable to use the monomethylene group.

As an example of halogen atoms, represented by X composing the general formula, an fluorine atom, a chlorine atom, a bromine atom, and an iodine atom can be cited.

As an example of substituted benzyl groups represented by R⁴, a diphenyl group, and a triphenylmethyl group can be cited.

When a ring is formed by uniting R² and R³ in the general formula (I), as an example of a desirable ring, cyclopropane, cyclobutane, cyclopentane, cyclohexane or the like can be cited.

The polymerization degree of a polymer or a copolymer having a repeated unit represented by the general formula (I) is acceptable if it is 2 or more in the number of repeated units, and the number of repeated units of 10 to 500 is preferable.

When the above-described temperature-responsive polymer layer 2 is immobilized to the magnetic particulate 1, it is preferable to give the following pretreatment to the magnetic particulate 1. In other words, by reacting the magnetic particulate 1 and a compound expressed by the following general formula (II) (Chemical Formula 3), it is desirable to react with a polymer or a copolymer containing an acrylamide derivative expressed by the above-described general formula (I) after introduction of a functional group using a silane coupling reagent.

[Chemical Formula 3] X'-R⁵-Si-Y'₃

Where in the general formula (II),
R⁵ represents a straight chain or a branched alkyl group with a carbon number of 1 to 6,
X' represents an amino group, an alkyl substituted amino group, a carboxyl group or -COOR⁶ (where R⁶ represents a straight chain or a branched alkyl group with a carbon number of 1 to 6, a phenyl group, a substituted phenyl group, a benzyl group or a substituted benzyl group), a hydroxyl group or a halogen group, and
Y' represents a halogen atom or an alkoxyl group.

In order to enhance the reactivity of the magnetic particulate 1 with a compound expressed by the general formula (II), it is effective to give a vitrification treatment to the magnetic particulate I. It is recommendable for such a vitrification treatment to carry out reaction of sodium silicate with hydrochloric acid, sulfuric acid, hydrogen peroxide or the like in the water and to dry them.

It should be noted that a dimethylamino group, a diethylamino group, a methylethylamino group, a trimethylamino group, dimethylethylamino group, triethylamino group, or the like can be cited as an example of the alkyl substituted amino groups represented by X' composing the general formula (II).

As a halogen atom represented by Y', a fluorine atom, a chlorine atom, a bromine atom and an iodine atom can be cited. As an alkoxyl group represented by Y, a methoxyl group and an ethoxyl group can be cited.

It should be noted that a solvent used for the reaction between the general formula (II) and the magnetic particulate is not limited in particularly, provided that it does not participate in the reaction, for instance, the following chemicals can be used. That is, water, methanol, ethanol, n-propanol, i-propanol, acetic acid, acetone, 2-butanone, tetrahydrofuran, dioxane, acetonitrile, benzene, toluene, xylene, chlorobenzene, chloroform, methylene chloride, carbon tetrachloride, dimethylformamide, dimethylacetamide, dimethylsulfoxyde, and the like. It is also possible to use a mixture consisting of two kinds or more selected from these solvents, provided that they are miscible with each other. The reaction is desirably carried out at 0°C to 100°C.

In a reaction to immobilize a polymer or a copolymer expressed by the general formula (I) to the magnetic particulate 1 with a covalent bond, it is sufficient that X or Y in the general formula (I) is reacted with X' expressed by the general formula (II) which is to be reacted with the magnetic particulate 1 in advance.

For instance, in order to easily proceed the reaction, if X or Y in the general formula (I) is a carboxyl group, it is preferable that X' in the general formula (II) be a hydroxyl group or an amino group. If X or Y in the general formula (I) is a hydroxyl group or an amino group, it is preferable that X' in the general formula (II) be a carboxyl group. In this case, especially, when X or Y in the general formula (I), or X in the general formula (II) is a carboxyl group, the reaction progresses quite easily if it is changed to a succin-imide group, p-nitrophenylester group, or the like. Even when such a change does not occur, the reaction can be accelerated by using a condensing agent. As the condensing agent, for instance, dicyclohexylcarbodiimide, diisopropylcarbodiimide, N-etyl-N'-3-dimethylaminopropylcarbodiimide, benzotriazole-1-yl-tris (dimethylamino) phosphonium hexafluorophosphate, and diphenylphospharylazide can be cited. These condensing agent can be used alone, or can be used in combination with N-hydroxysuccinimide, 1-hydroxybenzotriazole, or the like.

The reaction to immobilize the polymer or copolymer containing an acrylamide derivative expressed by the general formula (I) to the magnetic particulate 1 with a covalent bond is preferably carried out in a solvent which does not participate in the reaction. As such solvents, for instance, water, methanol, ethanol, n-propanol, i-propanol, acetic acid, acetone, 2-butanone, tetrahydrofuran, dioxane, acetonitril, benzene, toluene, xylene, chlorobenzene, chloroform, methylene chloride, carbon tetrachloride, dimethylformamide, dimethylacetoamide, dimethylsulfoxide, and the like. It is also possible to use a mixture consisting of two kinds or more selected from these solvents, provided that they are miscible with each other. The reaction is desirably carried out at 0°C to 100°C.

In order to smoothly proceed the reaction to immobilize the polymer or copolymer containing an acrylamide derivative expressed by the general formula (I) to the magnetic particulate 1 with a covalent bond, it is preferable to carry out the reaction under a basic condition, and in order to create such a basic condition, it is preferable to use potassium hydroxide, sodium hydroxide, potassium carbonate, sodium carbonate, pyridine, N, N-dimethyl pyridine, trimethylamine, triethylamine, triethanolamine or the like.

When the anticancer agent 3 is immobilized to a temperature-responsive polymer composing the temperature-responsive polymer layer 2, it is recommended that, for instance, after the temperature-responsive polymer layer 2 is formed around the magnetic particulate 1 as described above, the resultant mixture is dispersed in water dissolving the anticancer agent. By conducting such treatment, a carboxyl group or the like which does not participate in the reaction of the temperature-responsive polymer and the anticancer agent molecule voluntarily bond ionically to each other. At this time, there is no necessity to use chemicals in particular. However, when the solubility of the anticancer agent in water is insufficient, the solubility of the anticancer agent can be enhanced by mixing an organic solvent miscible in water (for instance, methanol, ethanol, i-propanol, n-butanol, dimethylformamide, dimethylacetamide, dimethylsulfoxide, acetone or the like) as a dissolution assistant with water. Note that the sort of the organic solvent is not limited to those described above, and the rate of the organic solvent is preferably 50% by volume or less to the total sum of the solution.

By conducting such treatment, the composite particulate relating to the above-described embodiment can be obtained. When cancer treatment is performed using this composite particulate, for instance, after locally positioning the composite particulate close to a cancer cell by local injection or the like, an alternating-current magnetic field at 100kHz to 10MHz is given by, for instance, a radio wave. As a result, the magnetic particulate 1 generates heat to the extent of 43°C, a local temperature near the cancer cell becomes high. Therefore, a thermal treatment can be conducted by keeping this temperature. In addition, a temperature-responsive polymer is contracted in response to the local high temperature, the anticancer agent 3 ionically bonding to a hydrated graft structure of the temperature-responsive polymer is gradually released and disengaged, and attacks the cancer cell so that a chemical treatment can be achieved. Accordingly, a thermotherapy and chemotherapy can be realized spontaneously.

The reaction between a compound expressed by the general formula (II) and the magnetic particulate 1 can be confirmed using an X-ray photoelectron spectroscope and an infrared spectrophotometer. Furthermore, the reaction between the magnetic particulate 1 reacted with the general formula (II) and a polymer or a copolymer containing an acrylamide derivative expressed by the general formula (I) can be confirmed using a transmission electron microscope.

The composite particulate according to the present invention is suitable for a thermo- and chemotherapy medicine such as a medicine for injection for thermo- and chemotherapy containing the composite particulate as an effective component. It is also suitable for a device for thermo- and chemotherapy such as a local injection device, an alternating-current imparting device, a microwave hyperthermia device, a short wave hyperthermia device or the like, provided with a position identifying device for a cancer cell.

Although there is a disadvantage that the sensitivity in response to a temperature is lowered when the functional groups are increased in the conventional temperature-responsive polymer, the temperature-responsive polymer expressed by the general formula (I) does not have such a disadvantage. It becomes possible to execute effective chemotherapy by using a composite particulate in which this temperature-responsive polymer is connected to a particulate. In other words, since it is possible to increase functional group while maintaining high sensitivity in temperature response, it becomes possible to give the required amount of antibodies. There is also an advantage that various substances can be used as functional groups. For instance, when a carboxyl group, a primary amino group, a secondary amino group, a tertiary amino group, a hydroxyl group, and the like are used, a sufficient effect can be obtained.

It is also possible to use, for instance, a metal particulate, an activated carbon particulate, or the like, other than the magnetic particulate.

Hereinafter, the samples actually prepared by the present inventor will be explained in detail. However, the present invention is not limited to these embodiments.

### -Embodiment 1 of the Present Invention-

First, 0.5 g of commercially available magnetic particles (average diameter: 150 nm) was dispersed in a mixed solvent of 12.5 ml acetic acid and 12.5 ml ethanol. Then, 3-aminopropyl trimethyl methoxysilane 0.5 ml was added to it and they were agitated for 18 hours at room temperature. Then, it was thoroughly cleansed with ethanol and dried.

Meanwhile a temperature-responsive polymer was prepared. Here, 11.25 g of 2-carboxyisopropyl acrylamide and 8.24 g of hydroxysuccinimide were dissolved in a mixed solvent of dioxane 120 ml and ethyl acetate 100 ml. 16.25 g of dicyclohexyl carbodiimide was added to this solution and allowed to react for one hour at 0°C, and then, for 15 hours at room temperature. The crystals thus obtained was dissolved in isopropanol and kept it for 24 hours at 4°C. Then, the deposited crystal was filtrated, and the residual was condensed in an evaporator and dried to obtain 2-carboxyisopropylacrylamide succin-imide ester.

Next, 2-carboxyisopropylacrylamide succinimide ester obtained as described above, isopropylacrylamide, azobisisobutyronitril as an initiator, and dimethylformamide as a solvent were put into a glass polymerization pipe in the respective amounts described in the following Table 1 and deaerated. Then, a polymerization reaction was conducted for 3.5 hours at 70°C. After the reaction was over, the polymerization product was precipitated in diethylether to recover the polymer. Then, the polymer was dissolved in tetrahydrofuran. The polymer was recovered by pouring the solution into diethylether again, and dried under a reduced pressure. Thus, the temperature-responsive polymer was prepared.

**[Table 1]**

| temperature-responsive polymer | 2-carboxy isopropylacryl amide succin imide ester | isopropyl acryl amide (g) | azobisisobutyro nitril (mg) | dimethyl formamide (ml) |
|---|---|---|---|---|
| No. 1 | 4.0 (g) | 15.96 (g) | 64.1 (mg) | 84.6 (ml) |
| No. 2 | 1.5 (g) | 12.63 (g) | 44.9 (mg) | 60.0 (ml) |

Then, the above-treated magnetic particulate 0.2 g and each 0.2 g of the temperature-responsive polymers No. 1 and No. 2 were dispersed and dissolved in a phosphate buffer solution containing 40 µl of triethylamine. Then, after agitating for 24 hours at 15°C, and cleansed with a large amount of cold water, two kinds of temperature-responsive polymer immobilizing magnetic particulates were prepared.

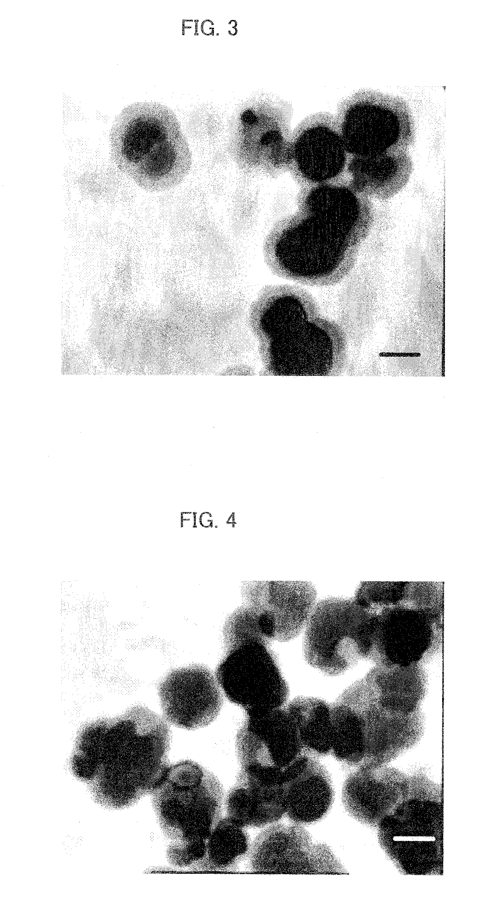

These two kinds of temperature-responsive polymer immobilizing magnetic particulates were placed on a collodion grid strained with a collodion film and was observed through a transmission electron microscope. The observed images are shown in Figs. 1 and 2. Fig. 2 is a TEM photo showing an image of the temperature-responsive polymer immobilizing magnetic particulate using the temperature-responsive polymer No. 1. Fig. 3 is a TEM photo showing an image of the temperature-responsive polymer immobilizing magnetic particulate using the temperature-responsive polymer No. 2. As shown in Figs. 2 and 3, it is confirmed that the temperature-responsive polymer is immobilized around the magnetic particulate so that the temperature-responsive polymer layer is formed. Accordingly, these temperature-responsive polymer immobilizing magnetic particles can easily immobilize a compound such as an anticancer agent or the like.

### -Comparison Example 1-

A commercially available untreated magnetic particulate (average diameter: 150 nm) was placed on a collodion grid strained with a collodion film and was observed through a transmission electron microscope. The observed image is shown in Fig. 4. As shown in Fig. 4, nothing was observed on the surface of the magnetic particulate.

### -Reference Example 1-

When a magnetic particulate is reacted with 3-aminopropyl-trimethylmethoxysilane, the following treatment may be conducted. That is, a commercially available magnetic particulate (average diameter: 150 nm) 0.5g is dispersed in the solvents described in Table 2 below. Then, 3-aminopropyltrimethyl-methoxysilane 0.5ml is added and agitated for 18 hours at room temperature or at 80°C. After that, it was thoroughly cleansed with ethanol, and allowed to dry.

**[Table 2]**

| Reference Example | Solvent 1 | Amount of Solvent 1 (ml) | Solvent 2 | Amount of Solvent 1 (ml) | Temperature |
|---|---|---|---|---|---|
| 1 | acetic acid | 25 | none | none | room temperature |
| 2 | ethanol | 24 | water | 1 | room temperature |
| 3 | ethanol | 24 | water | 1 | 80°C |
| 4 | toluen | 25 | none | none | room temperature |

As will be described below, a magnetic particulate may be reacted with 3-aminopropyl-trimethylmethoxysilane after vitrification treatment of the magnetic particulate. That is, the commercially available magnetic particulate (average diameter: 150 nm) 0.2g is dispersed in an aqueous solution containing hydrogen peroxide and sodium silicate described in Table 3 below. Next, after stirring for 8 hours at 80°C, it was cleansed with a large amount of distilled water and ethanol, and dried under a reduced pressure.

**[Table 3]**

| Reference Example | hydrogen peroxide | sodium silicate (mg) | distilled water (ml) |
|---|---|---|---|
| 5 | 1 | 240 | 7.5 |
| 6 | 3.3 | 240 | 7.5 |
| 7 | 3.5 | 240 | 5.2 |

Then, 50 mg of the magnetic particulate thus vitrification treated is taken by measure and dispersed in the solvents described in Table 4 below. After that, 3-aminopropyl-trimethylmethoxysilane 0.1 ml is added and it is continued to agitate for 18 hours at room temperature. Then, it is thoroughly cleansed with ethanol and dried.

**[Table 4]**

| Reference Example | Verification Treated Magnetic Particulate | Solvent 1 | Amount of Solvent 1 (ml) | Solvent 2 | Amount of Solvent 2 (ml) |
|---|---|---|---|---|---|
| 8 | Reference Example 5 | acetic acid | 2.5 | ethanol | 2.5 |
| 9 | Reference Example 5 | ethanol | 2.5 | water | 2.5 |

### -Embodiment 2 of the Present Invention-

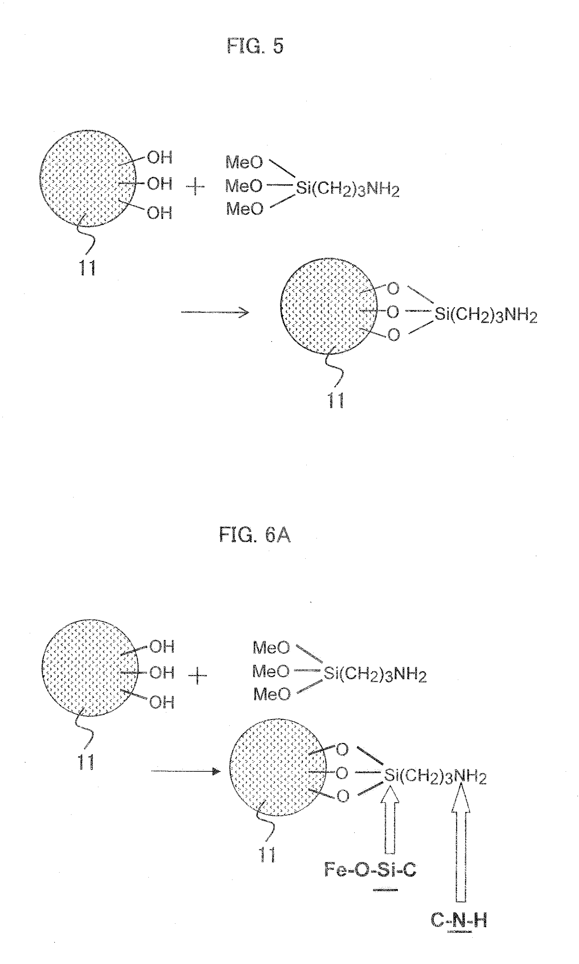

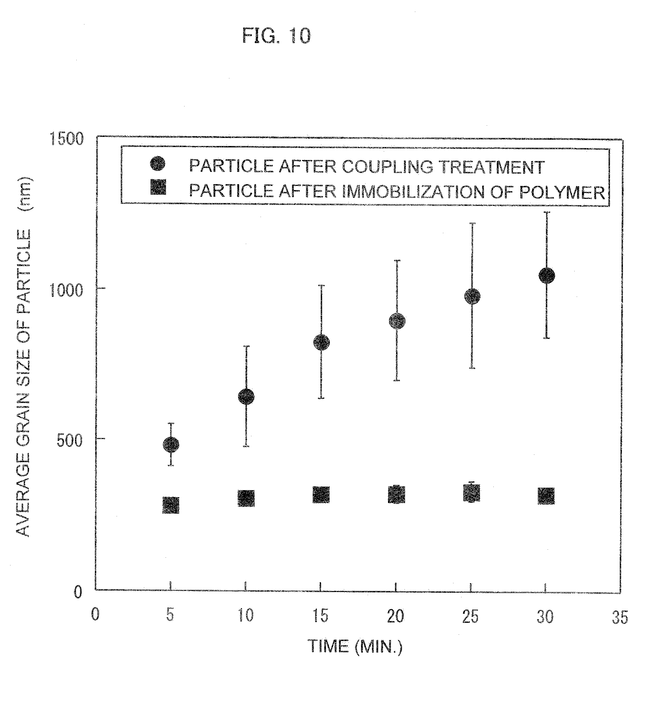

In the present embodiment, introduction of an amino group to the surface of the particulate by silane coupling treatment was first conducted. In this introduction, first, 2g of magnetite particulate 11 was dispersed in 400 ml of solvent, which was then subjected to ultrasonic treatment for 30 minutes. A mixed solution of acetic acid and ethanol at a ratio of 1:1 by volume was used as the solvent. Then, as shown in Fig. 5, 10 ml of 3-aminopropyl-trimethylmethoxysilane was added to this solvent as a silane coupling reagent, and allowed to react for 24 hours at room temperature. After that, it was cleansed 5 times with distilled water. Then, it was substituted with ethanol. After drying it for 24 hour under a reduced pressure, the particulate was recovered. Note that the hydroxyl group attached to the magnetite particulate 11 in Fig. 5 is a portion of iron hydroxide.

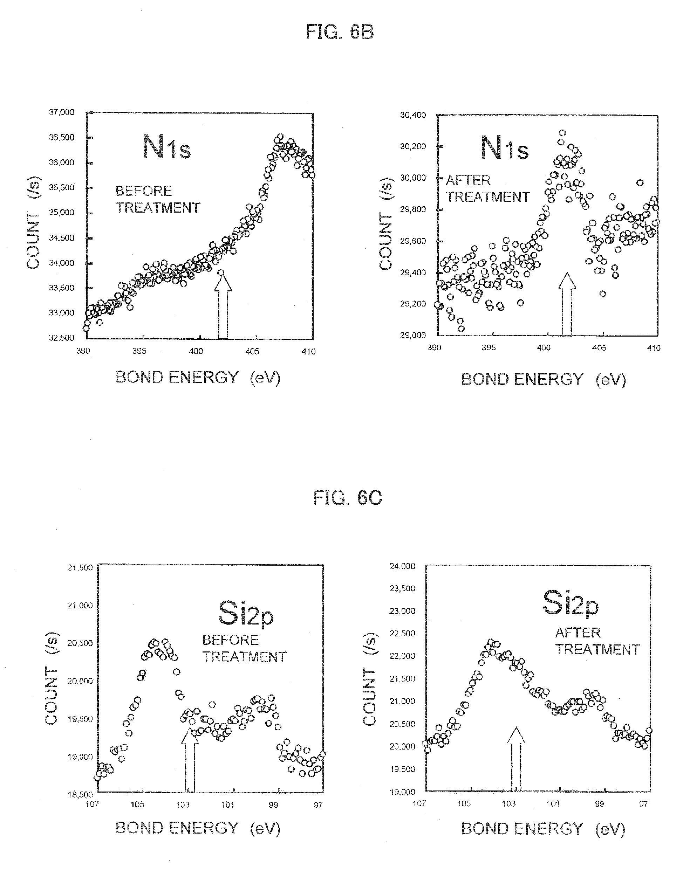

The introduction of the silane coupling reagent was evaluated using an X-ray photo electron spectroscopy (XPS). In this evaluation, before and after the above-described silane coupling processes (Fig. 6A), the spectroscopies of nitrogen (N) 1s orbit and that of the silicon (Si) 2p orbit were studied. As a result, as shown in Fig. 6B, a new peak was observed in the nitrogen (N) 1s orbit after the silane coupling process. This peak is resulted from the amino group of the silane coupling reagent. It was also observed as shown in Fig. 6C that the peak of the silicon (Si) 2p orbit was shifted just before and after the reaction. The reason of the shift is considered to come from the formation of chemical bonding between the hydroxyl group (-OH) on the surface of the particulate and a silicon ion (-Si) of the silane coupling reagent. Accordingly, it can be said that the silane coupling reagent is introduced to the surface of the magnetite particulate by the method described above.

The immobilization of polymer (temperature-responsive polymer) to a collected particle was conducted next. In the immobilization, the particulate 0.2g was dispersed in a polymer solution first, ultrasonic treatment was performed for 30 minutes in ice. The polymer solution was prepared by dissolving 0.5g of a polymer in 5 ml of water. The polymer was prepared as follows. That is, isopropyl acrylamide 30.54g, 2-carboxylisopropylacrylamide 0.539g and azo-bis-isobutyronitril 0.033g were dissolved in 10 ml of dimethylformamide, put into a glass polymerization tube, and deaerated. Then, a polymerization reaction was conducted for 24 hours at 60°C. After the reaction was over, the reaction solution was put in a dialysis tube having a molecular weight of 3500, and purification was carried out by performing dialysis against water for 5 days while keeping at 4°C.

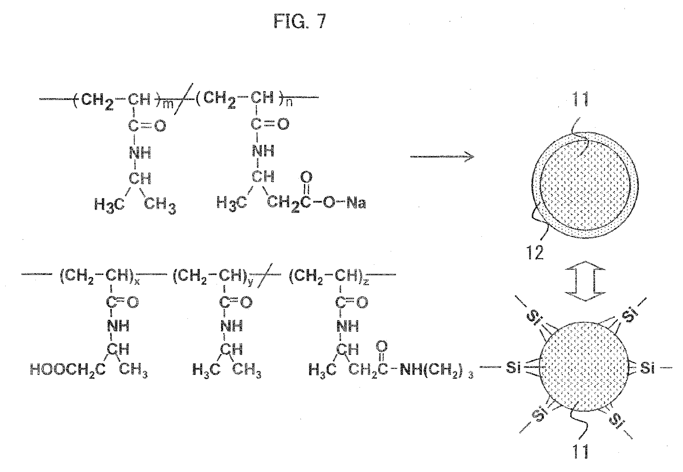

After the above-described ultrasonic treatment, about 44.67 mg of water soluble carbodiimide (WSC) serving as a condensing agent was added to the polymer solution. Note that about 44.67 mg of water soluble carbodiimide corresponds to 0.5 times as much as the amount of the carboxyl group content of the polymer. Then, the ultrasonic treatment was conducted for one hour in ice. It was kept for 24 hours at 4°C. As a result, a temperature-responsive polymer layer 12 was formed as shown in Fig. 7. The temperature-responsive polymer 12 is chemically immobilized to the magnetite particulate 11 via silicon (Si). Then, cleansing with water is conducted five times or more to remove the residual polymer. Then, after sufficient cleansing, it was substituted with ethanol. Then, after drying under a reduced pressure, the composite particulate was collected. It should be noted that centrifugal separation for 30 minutes at 3000 revolutions per minute (rpm) is effective for the cleansing.

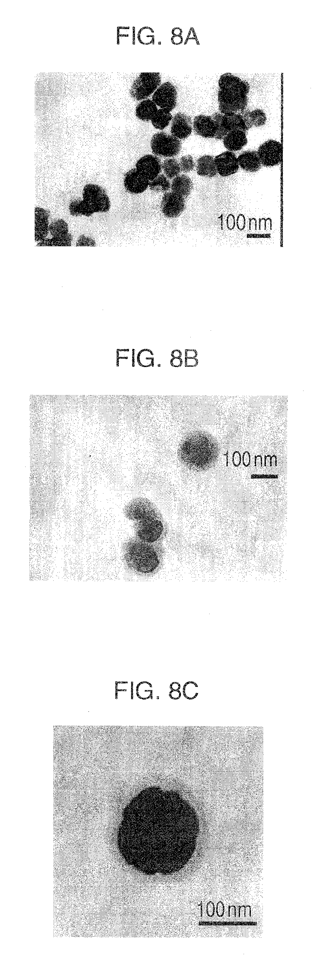

Here, a TEM observation of the composite particulate was conducted. In the TEM observation, the composite particulate after immobilization of polymer was dispersed in water and a pattern observation using TEM was performed. The particulate before immobilization of the polymer was observed for reference. As a result, as shown in Fig. 8A, in the TEM photo of the particulate before the polymer immobilization, only black magnetite particles were observed, but as shown in Figs. 8B and 8C, in the TEM photo of the composite particulate after the polymer immobilization, a gray layer around the black particulate was observed. The thickness of this gray layer was 10nm to 20nm. This layer is considered to be originated from the polymer immobilized on the surface of the magnetite particle 11.

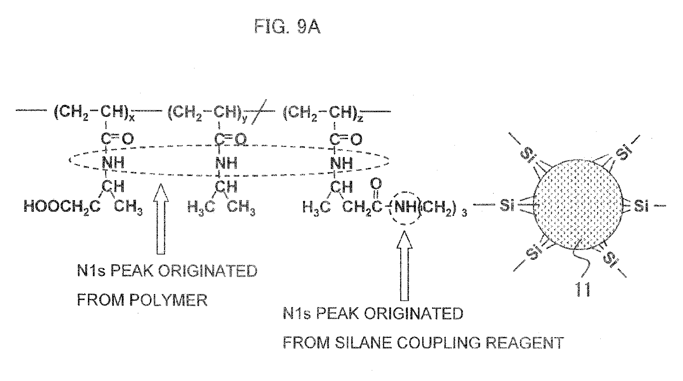

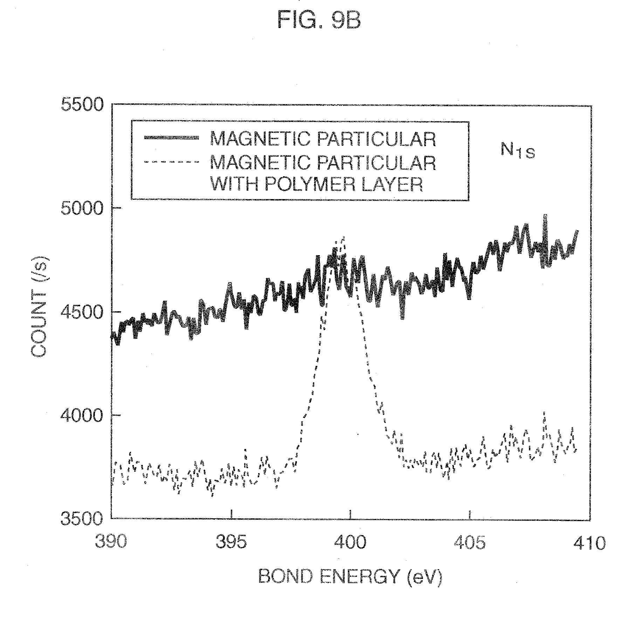

Evaluation of the polymer immobilization was conducted using an X-ray photoelectron spectroscopy (XPS). In this evaluation, an analysis of the nitrogen (N) 1s orbit was conducted before and after the above-described polymer immobilization (Fig. 9A). Note that there are nitrogen (N) originated from the silane coupling agent and that originated from the polymer in N as shown in Fig. 9A. However, since the nitrogen (N) originated from the silane coupling reagent exists in a very small quantity compared with that originated from the polymer, it can be understood that the peak appeared is based on nitrogen (N) originated from the polymer. As a result of this analysis, as shown in Fig. 9B, in the composite particulate after the polymer immobilization, a distinct peak was observed in the nitrogen (N) 1s orbit in the composite particulate after the polymer immobilization. This peak is caused by the existence of an amide bond originated from the polymer. From this, it can be said that the polymer is immobilized on the surface of the composite particulate.

In addition, evaluation of the relation between immobilization of polymer and dispersion stability was conducted. In this evaluation, coagulation behavior of the particulate was observed using Dynamic Light Scattering (DSL) measurement. The composite particulate after the polymer immobilization was first dispersed in water in concentration of 0.1 mg/ml or below, and was subjected to ultrasonic treatment for 30 minutes. Next, ultrasonic treatment is conducted for 3 minutes, and measurement of the particle diameter was started. The change in average particle size as time passes was observed. The temperature at this time was set at 20°C or below, which is the Lower critical Solution Temperature (LCST) of the polymer. It is possible to take the lower critical solution temperature (LCST) as a phase transition temperature. For reference, the similar observation was conducted for the particulate after silane coupling treatment before performing the polymer immobilization. As a result, a distinct increase in particle size accompanying the progress of time was observed in the particulate (•) with the silane coupling treatment only, as shown in Fig. 10. This is considered to be due to progress of coagulation of the particulate because the influence due to magnetic interaction and coagulation, effect between magnetite particulates are stronger than the improvement in dispersibility accompanying the immobilization of the silane coupling reagent. Whereas, in the composite particulate (■) after the polymer immobilization, increase in particle size accompanying the progress of time was not observed. This is considered to be because the dispersibility is enhanced more than the coagulation effect occurring between the particles due to hydration of the immobilized polymer.

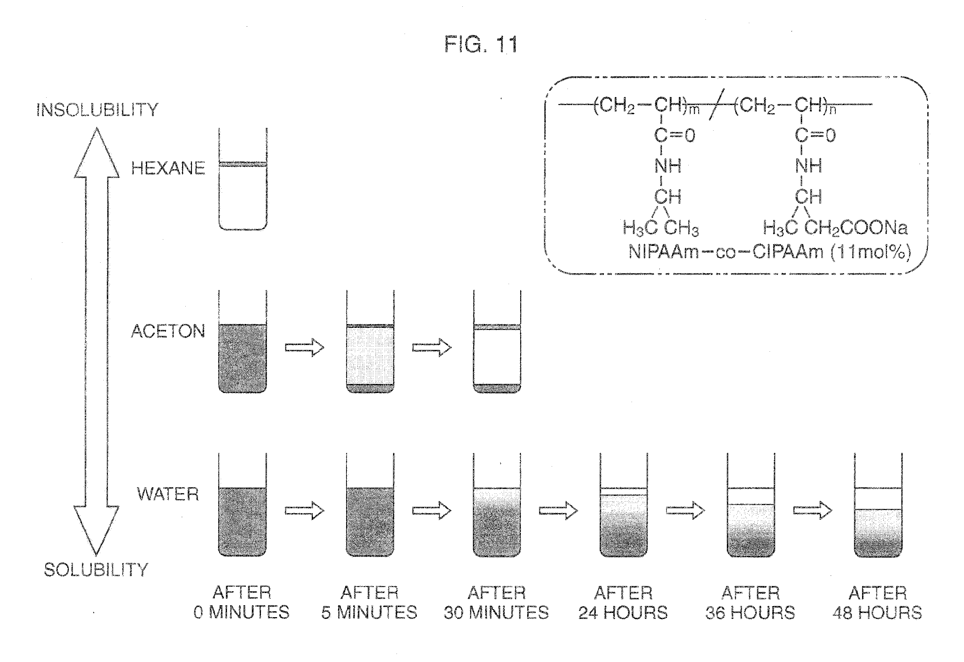

In addition, evaluation of the dispersibility accompanying solvent affinity of the polymer was conducted. First, the composite particulate was added to the solvent so as to be in concentration of 1 mg/ml. Then, ultrasonic treatment for 3 minutes was conducted and it was observed after leaving it at room temperature. Hexane, acetone, and water to which the solvent affinities of the polymer are different, are used as solvents. As a result, as shown in Fig. 11, it is observed that the higher the polymer affinity the solvent has, the higher dispersibility it shows. Note that the chemical formula at the right above in Fig. 11 shows a rigorous structure of the polymer at the time of evaluation. That is, hydrogen (H) in the carboxyl group was substituted by nitrogen (N) at the time of evaluation. As a result, though the composite particulate could disperse in water very easily, it did not disperse in hexane at all. Furthermore, the composite particulate could hardly disperse in acetone. It should be noted that when hydrogen (H) in the carboxyl group is not substituted, the polymer is easily soluble in acetone, which should give a different result.

From the above circumstance, a polymer is said to be immobilized on the surface of the composite particulate. In addition, it can be said that in the composite particulate, the solvent affinity of polymer largely contributes to the dispersibility of the composite particulate.

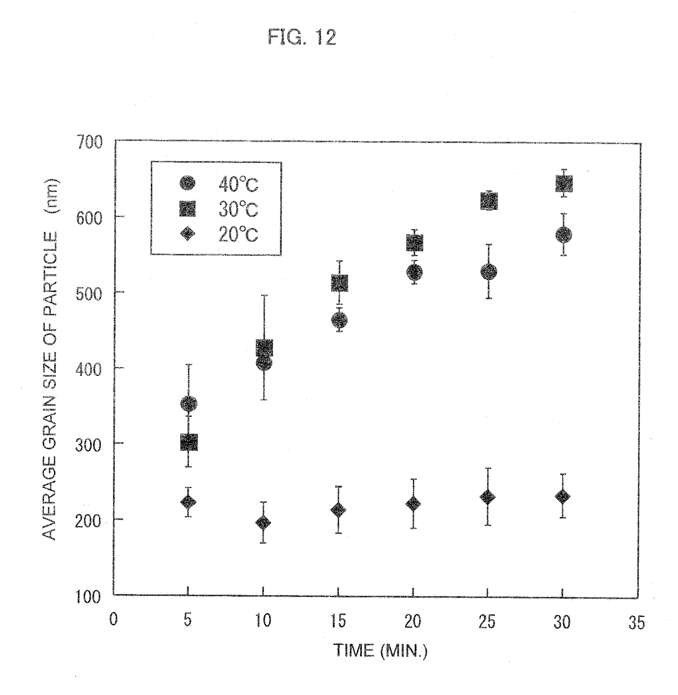

Furthermore, observation of the phase transition behavior of the composite particulate after polymer immobilization was conducted. Here, a relation between temperature change and the coagulation behavior of the composite particulate was observed. In this observation, similarly to the evaluation of dispersion stability accompanying the polymer immobilization, the coagulation behavior of the particulate was observed by means of DLS measurement. First, the composite particulate after polymer immobilization was dispersed in a solvent in concentration of 0.1 mg/ml or lower, and ultrasonic treatment was conducted for 30 minutes. As the solvent, 100mM NaCl aqueous solution was used. The LCST of the 100mM NaCl aqueous solution containing the polymer only was about 28°C. Then, it was kept at the respective measurement temperatures (20°C, 30°C and 40°C) for 30 minutes. Next, ultrasonic treatment was conducted for 3 minutes and measurement of the particle size was started. Then, variation of the average particle size accompanying the progress of time was observed. As a result, as shown in Fig. 12, a distinct difference appeared in the dispersion stability of particle above and below the LCST (phase transition temperature) of the polymer. It is considered that since phase transition appears in the polymer immobilized on the surface of the composite particulate and becomes hydrophobic, which promotes a coagulation force between composite particulates.

It should be noted that similar behavior was observed also in pure water though its temperature was different due to variation of phase transition temperature of the polymer. However, the variation in the average particle diameter was more remarkable in the case of using 100 mM NaCl aqueous solution.

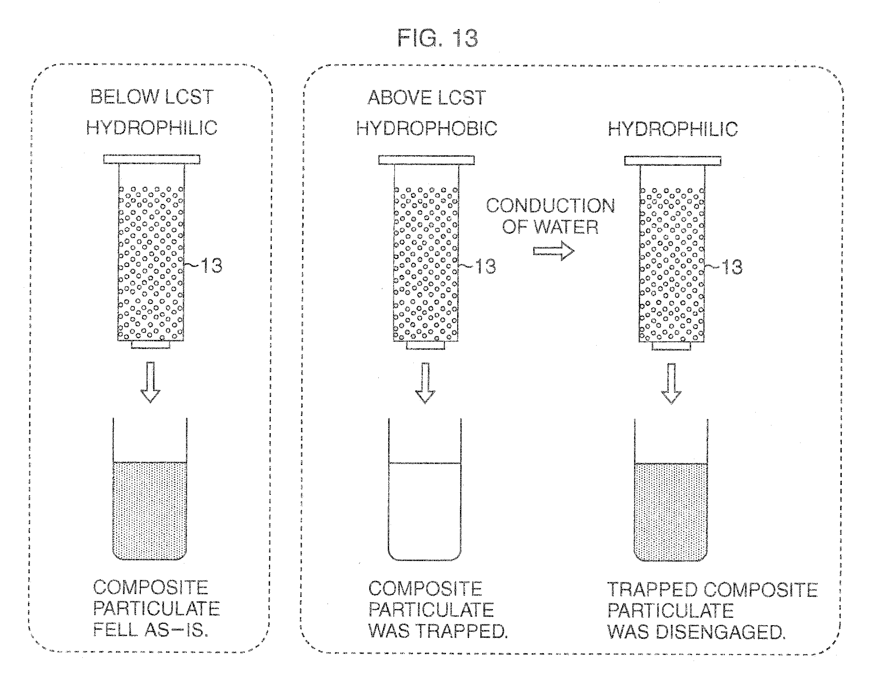

Furthermore, an adsorption experiment using a hydrophobia silica particulate having an octadesyl group (ODS) was conducted to observe how the surface of the composite particulate becomes hydrophobic. In the observation, whether the surface of the polymer immobilized, composite particulate became hydrophobic was confirmed using an ODS column in which a silica particulate (ODS) processed with a hydrophobic silane coupling reagent was packed. First, two kinds of solutions in which the composite particulate was dispersed in the concentration of about 0.1 mg/ml were prepared and they were kept for 30 minutes at 55°C and 20°C respectively. The temperature of 55°C is obviously above the phase transition temperature (LCST) of the polymer, and the temperature of 20°C is obviously below the phase transition temperature. The temperatures of the solutions were made stable by this setting. The temperature inside a column 13 was adjusted to be nearly at the measurement temperature by flowing water inside the column 13 at the measurement temperatures of 55°C and 20°C. When the solution (dispersion of the composite particulate) was allowed to pass through under such conditions, the composite particulate passed through the column at 20°C (below LCST), but was trapped inside the column at 55°C (above LCST) and hardly passed through the column, as shown in Fig. 13. The reason why the composite particulate hardly passed through is considered to be due to hydrophobic mutual effect accompanying hydrophobic formation of the composite particle surface. When 20°C water was let flow inside the column in which the composite particulate was trapped, the trapped composite particulate was flown out. It is considered that since the temperature of the composite particulate became below LCST, which makes the surface of the composite particulate bear hydrophilic again.

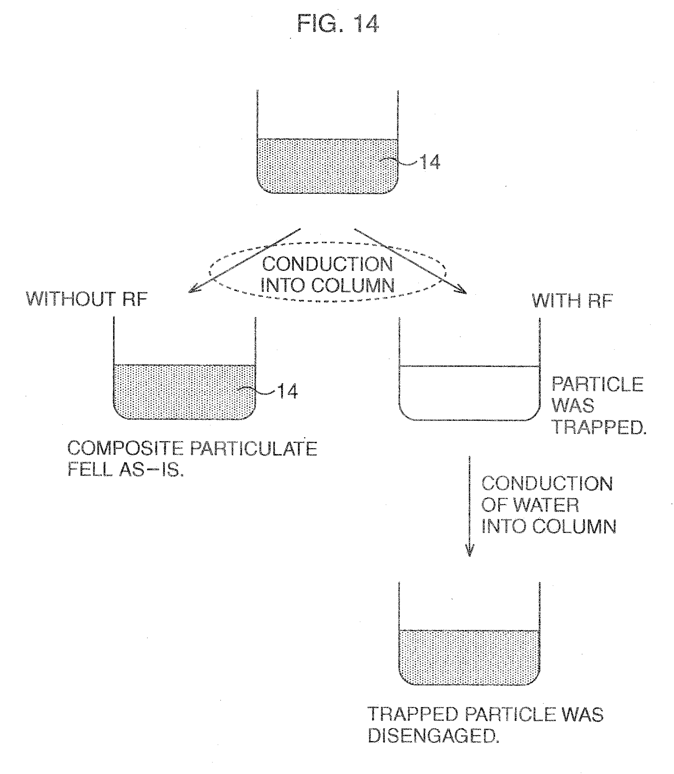

Further, phase transition behavior by self-heat generation of the composite particulate using an RF radiation was observed. Here, possibility of phase transition of the polymer immobilized on the surface of the magnetite particulate by self-heat generation (originated by a hysteresis loss) of the composite particulate accompanying irradiation of an alternating-current magnetic field was confirmed. In this observation, the measurement temperature was set to be 20°C, a solution was prepared in a similar manner to that in the above-described adsorption experiment. The conditions of the alternating-current magnetic field were determined to be frequency 300kHz, output 1kW, and the coil winding number being 12 turns. The ODS column was inserted into the coil and the solution was passed through the ODS column. At this time, by circulating water in the coil at 20°C, temperature increase due to heat generation of the coil itself was restrained. As a result, as shown in Fig. 14, when the RF radiation was not irradiated, the composite particulate in the solution 14 passed through the column, but when the alternating-current magnetic field was irradiated, the composite particulate was trapped in the column. It is considered that the reason why the composite particulate was trapped in the column is because the surface of the composite particulate becomes hydrophobic. In other words, it can be said that generation of phase transition of the polymer immobilized on the surface of the composite particulate is possible by means of self-heat generation of the composite particulate caused by RF irradiation. The composite particulate trapped in the column could be disengaged from inside the column by passing water through.

In addition, immobilization of anticancer agent to the composite particulate and the confirmation thereof were conducted. Here, immobilization of electrostatic doxorubicine to a carboxyl group of a polymer constituting the temperature-responsive polymer layer 12 was conducted. First, the composite particulate was dispersed in cold water in concentration of 0.5 mg/ml. Next, ultrasonic treatment was conducted for 10. minutes. Then, doxorubicine was dissolved in concentration of 0.05 mg/ml. Thereafter, ultrasonic treatment was conducted for 10 minutes to react it for 3 hours at 4°C. Then, centrifugal separation was conducted for 15 minutes at a rotational speed of 12,000 rpm to collect supernatant liquor. Then, measurement of ultraviolet absorption luminosity (absorption spectrum) of doxorubicine was conducted. As samples, doxorubicine only (sample A), a mixture of doxorubicine and the composite particulate to which WSC amounting 0.5 times as much as that of the composite particulate was added (sample B), a mixture of doxorubicine and the composite particulate to which WSC amounting 1.0 times as much as that of the composite particulate was added (sample C), a mixture of doxorubicine and the composite particulate to which WSC amounting 1.5 times as much as that of the composite particulate was added (sample D), and a mixture of doxorubicine and unprocessed magnetite particulate (sample E) were used.

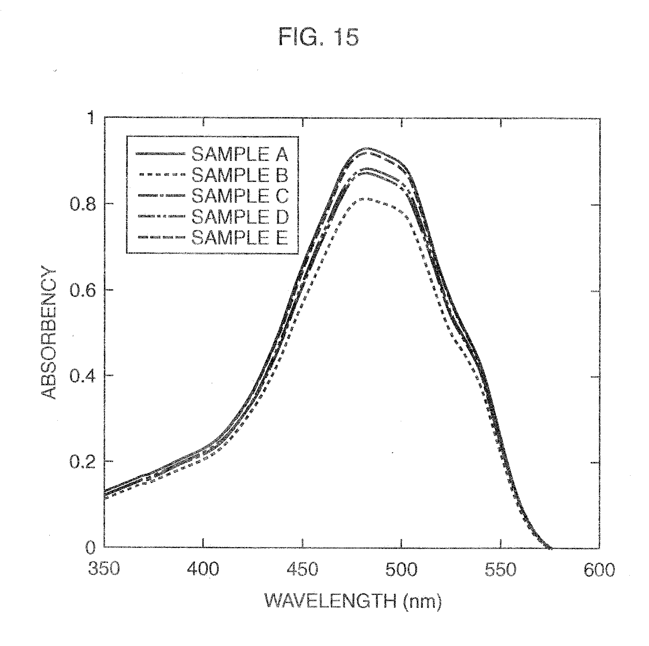

As a result, as shown in Fig. 15, there is no difference between absorption spectrum of the mixture of doxorubicine with unprocessed magnetite particulate (sample E), and that of doxorubicine only (sample A). Therefore, immobilization of doxorubicine to the magnetite particulate was not confirmed. Whereas, in the mixtures of doxorubicine and the composite particulate (sample B to sample D), absorption spectra differ from each other. This is considered that since doxorubicine was immobilized to the composite particulate, residue in the solution is reduced. When the sample having the amount of addition of WSC to be 1.0 time or 1.5 times as much as that of the composite particulate (samples C and D) was compared with the sample having the amount of addition of WSC to be 0.5 times as much as that of the composite particulate (sample B), the absorption spectrum was found to be remarkably reduced in sample B. The reason is considered to be because a condensation reaction between the polymer and the magnetite particulate is promoted in samples C and D, whereas the content of carboxyl groups remaining in the polymer is larger in sample B. From this, it can be said that the carboxyl group in the polymer electrostatically reacts to doxorubicine. Further, it can be said that immobilization of the polymer on the surface of the magnetite particulate is carried out based on chemical bonding through a condensation reaction. In addition, it can be said that more doxorubicine can be immobilized by making the concentration of the composite particulate high.

Furthermore, an experiment of releasing doxorubicine immobilized on the particulate surface accompanying temperature variation was conducted. In the experiment, the behavior of doxorubicine electrostatically immobilized on the surface of the composite particulate to be released accompanying the phase transition of the polymer was observed. Here, the above-described sample E was used. After the sample E was kept for one hour at 4°C, centrifugal separation was carried out at 4°C. Then, its supernatant liquor was collected to measure ultraviolet absorption luminosity (absorption spectrum) of doxorubicine.

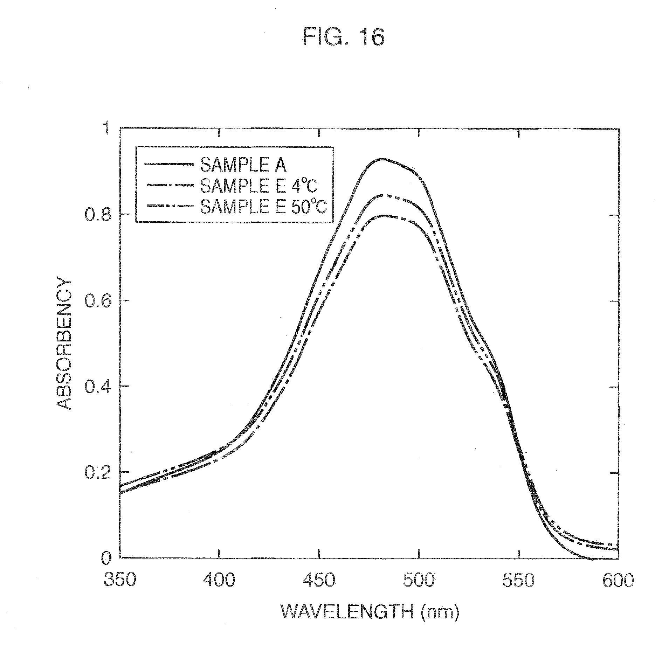

As a result, as shown in Fig. 16, the absorption spectrum of doxorubicine was reduced. When the temperature of the solution was raised to 50°C after confirmation of immobilization of doxorubicine, the absorption spectrum showed recovery from the reduction by about 40% of reduced amount caused by the immobilization. From this fact, it can be said that electrostatically immobilized doxorubicine is likely to be released accompanying the phase transition of the polymer.

It should be noted that immobilization of a stimulation-responsive polymer to a magnetic particulate is described in Patent Document 1, and the usage described in it is to easily recover a magnetic particulate having a particle diameter of 1 µm or less according to the property of the stimulation-responsive substance. However, there is no description nor suggestion of the release of a compound such as an anticancer agent or the like accompanying heat generation by a magnetic particulate.

### Industrial Applicability

As described above, according to the present invention, it is possible to introduce many functional groups while obtaining high sensitivity to the temperature variation of a temperature-responsive polymer. Therefore, it is suitable for an effective chemical therapy.

Since a functional substance is released from a hydrated graft structure accompanying heat generation of a magnetic particulate due to application of an alternating-current magnetic field, when the magnetic particulate which generates heat in the alternating-current magnetic field is used as a particulate, it is possible to send the functional substance to a desired position. Accordingly, when an anticancer agent is used as the functional substance, it is possible not only to release the anticancer agent to a cancer cell, but also to carry out a thermal treatment by heat generation of the magnetic particulate.

## Claims

1. A composite particulate, comprising:
a particulate; and
a hydrated graft structure connected to a surface of said particulate and composed of a temperature-responsive polymer and water,
wherein a general formula of said temperature-responsive polymer is expressed by 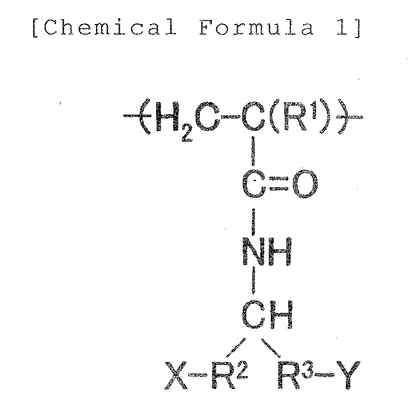 (where R¹ represents a hydrogen atom, or a straight chain or a branched alkyl group with a carbon number of 1 to 4,
R² represents a straight chain or a branched alkyl group with a carbon number of 1 to 4,
R³ represents a methylene group with a carbon number of 1 to 6,
X represents a hydrogen atom, an amino groups a hydroxyl group, a halogen atom, a carboxyl group or - COOR⁴ (where R⁴ represents a straight chain or a branched alkyl group with a carbon number of 1 to 6, a phenyl group, a substituted phenyl group, a benzyl group or a substituted benzyl group), and
Y represents an amino group, a hydroxyl group, a halogen atom, a carboxyl group or -COOR⁴ (where R⁴ represents a straight chain or a branched alkyl group with a carbon number of 1 to 6, a phenyl group, a substituted phenyl group, a benzyl group or a substituted benzyl group). It should be noted that R² and R³ may be united together and form a ring.).

2. The composite particulate according to claim 1, wherein said particulate is a magnetic particulate generating heat in an alternating-current magnetic field.

3. The composite particulate according to claim 2, wherein said particulate is connected with a silane coupling reagent on the surface of the particulate, and said hydrated graft structure is connected to a surface of said particulate via said silane coupling reagent.

4. The composite particulate according to claim 3, wherein a general formula of said silane coupling reagent is expressed by
[Chemical Formula 2] X'-R⁵-Si-Y'₃
(where R⁵ represents a straight chain or a branched alkyl group with a carbon number of 1 to 6,
X' represents an amino group, an alkyl substituted amino group, a carboxyl group or -COOR⁶ (where R⁶ represents a straight chain or a branched alkyl group with a carbon number of 1 to 6, a phenyl group, a substituted phenyl group, a benzyl group or a substituted benzyl group), a hydroxyl group or a halogen group, and
Y' represents a halogen atom or an alkoxyl group).

5. The composite particulate according to claim 4, further comprising a functional substance connected to said hydrated graft structure.

6. The composite particulate according to claim 5, wherein said functional substance ionically bonds to said hydrated graft structure.

7. The composite particulate according to claim 6, wherein said functional substance is an anticancer agent.

8. A method of manufacturing a composite particulate, comprising the steps of
introducing a functional group to a surface of a magnetic particulate using a silane coupling reagent;
connecting a hydrated graft structure composed of a temperature-responsive polymer and water to a surface of said magnetic particulate with a covalent bond via said functional group; and
connecting said hydrated graft structure and a compound with an ionic bond.

9. A medicine comprising the composite particulate according to any one of claims 1, 2, 3, 4, 5, 6 and 7 as an effective component.

10. The medicine according to claim 9, wherein said medicine is used for conducting a thermo-and chemotherapy for cancer.

11. A device for thermo-and chemotherapy, comprising:
a locally injector provided with a locator specifying a position of a cancer cell; and
an alternating-current magnetic field applying portion applying an alternating-current magnetic field at 100kHz to 10MHz to the composite particulate according to any one of claims 2, 3, 4, 5, 6 and 7 localized by said locally injector.
